# EUROPEAN PATENT APPLICATION

(11) **EP 4 517 770 A1**
(43) Date of publication of application: **05.03.2025**
(21) Application number: 23199054.0
(22) Date of filing: 22.09.2023
(51) Int. Cl.: G16H 30/40, G06T 7/00, G16H 40/63, G16H 50/20, G16H 50/70, G06N 3/02

(54) **IMAGE QUALITY IN CLINICAL SCANS**

(30) Priority: 30.08.2023 WO PCT/CN2023/115684
(71) Applicant: Koninklijke Philips N.V., 5656 AG Eindhoven (NL)
(72) Inventor: JIN, Peijie, Eindhoven (NL); DESHPANDE, Hrishikesh Narayanrao, Eindhoven (NL); JIN, Hua, 5656AG Eindhoven (NL); BUELOW, Thomas, Eindhoven (NL); SCHMITT, Holger, Eindhoven (NL); HARDER, Tim Philipp, Eindhoven (NL)
(74) Representative: Philips Intellectual Property & Standards

(57) **Abstract**

A method of guiding clinical scan quality assessments. The method comprises identifying one or more target anatomies in a region of interest of a clinical scan of a subject and determining an indication of regional image quality for each identified target anatomy.

## Description

### FIELD OF THE INVENTION

The invention relates to the field of image quality assessments of clinical scans.

### BACKGROUND OF THE INVENTION

Image quality plays an important role in correctly diagnosing medical images for the specific clinical target. Diagnostic medical images and radiology workflows can be severely impacted if the image quality does not meet the standard guidelines for a specific diagnostic target.

Different image quality issues give rise to unexpected effects of blurring, double images, or streaks in the reconstructed image. These artifacts appear in a variety of forms in diagnostic images. They could affect the imaging workflow in several ways, including increasing the possibility of wrong diagnosis, inefficient use of the scanner time before patient leaves, increasing radiation/dose exposure for rescan and sub-optimal utilization of clinician's source.

Thus, there is a need for a method which enables clinicians to accurately assess the quality of a clinical scan and decide if it is required to perform an additional (partial) rescan or not.

### SUMMARY OF THE INVENTION

The invention is defined by the claims.

According to examples in accordance with an aspect of the invention, there is provided a computer-implemented method of guiding clinical scan quality assessments, the method comprising:
identifying one or more target anatomies in a region of interest of a clinical scan of a subject; and
for each identified target anatomy, determining an indication of regional image quality.

The indications of regional image quality correspond to target anatomies. As such, an image quality metric is provided for localized portions in the region of interest as opposed to providing a global quality metric for the whole clinical scan.

This provides localized image quality information to the clinician to enable a more informed decision as to whether a re-scan is needed. This can reduce the time the subject spends in the scanner as well as reduce the amount of radiation the subject is exposed to (e.g., in computed tomography, CT, scans). Additionally, this can reduce the decision/review time for the user and ensure the image quality during image acquisition, especially under the high throughput of subjects.

The method may further comprise displaying a portion of the clinical scan corresponding to the region of interest overlayed with the indications of regional image quality.

The method may further comprise displaying subject information relating to the subject and/or diagnostic information indicative of a potential diagnosis for the subject.

Such a visualization can be helpful for clinicians to train on scan quality assessments as the indications of regional image quality are provided, thereby providing an intuitive sense of localized image quality.

Additionally, this can also provide more confidence and understandability for users as a timely quality assistant. In an example, the clinical information of the user (e.g., extracted by an NLP) and/or suggestions/actions may be displayed.

The method may further comprise determining the region of interest by extracting a diagnostic target from medical notes of the subject, determining possible causes for the diagnostic target and determining the region of interest based on the possible causes.

Medical notes can be used to determine the region of interest. A natural language processor (NLP) can be used to extract a diagnostic target (i.e., a target diagnostic) from the medical notes. A clinical knowledge database can then be used to infer possible causes for the diagnostic target. From the possible causes, a region of interest can be determined.

The method may further comprise determining a scan quality score for the clinical scan by combining the indications of regional image quality.

The indications of regional image quality can be combined to generate a global scan quality score for the whole clinical scan. The scan quality score will be biased towards the image quality of important anatomies as it is based on the indications of regional image quality determined for target anatomies in the region of interest, as opposed to using the image quality of the whole scan.

The scan quality score may be referred to as an overall quality score.

The method may further comprise, based on the scan quality score, classifying the clinical scan into suitable or unsuitable for diagnostic use.

The classification can be based on a pre-determined threshold for the scan quality score. The pre-determined threshold may be user adjustable.

The indication of regional image quality may comprise one or more of a detection of an artifact, a type of artifact identified, a complexity of identifying the corresponding target anatomy and blurring of the target anatomy.

Other indications of regional image quality may be used depending on the application (e.g., contrast, incomplete coverage etc.).

The method may further comprise inputting the clinical scan into a neural network configured to output one or more of: the detection of an artifact, the type of artifact identified, the complexity of identifying the corresponding target anatomy and blurring of the target anatomy.

The neural network may be trained to output said indication of image quality only for the region of interest.

The region of interest may be user adjustable.

The invention also provides a computer program carrier comprising computer program code which, when executed on a computer, causes the computer to perform all of the steps of the aforementioned method.

The computer program carrier may comprise computer memory (e.g., random-access memory), computer storage (e.g., hard drives, solid-state drives etc.). The computer program carrier may be a bitstream carrying the computer program code.

The invention also provides a system of guiding clinical scan quality assessments, the system comprising a processor configured to:
identify one or more target anatomies in a region of interest of a clinical scan of a subject; and
for each identified target anatomy, determine an indication of regional image quality.

The processor may be further configured to display a portion of the clinical scan corresponding to the region of interest overlayed with the indications of regional image quality.

The processor may be further configured to display subject information relating to the subject and/or diagnostic information indicative of a potential diagnosis for the subject.

The processor may be further configured to determine the region of interest by extracting a diagnostic target from medical notes of the subject, determining possible causes for the diagnostic target and determining the region of interest based on the possible causes.

The processor may be further configured to determine a scan quality score for the clinical scan by combining the indications of regional image quality.

The processor may be further configured to classify the clinical scan into suitable or unsuitable for diagnostic use based on the scan quality score.

The indication of regional image quality may comprise one or more of a detection of an artifact, a type of artifact identified, a complexity of identifying the corresponding target anatomy and blurring of the target anatomy.

The processor may be further configured to input the clinical scan into a neural network configured to output one or more of: the detection of an artifact, the type of artifact identified, the complexity of identifying the corresponding target anatomy and blurring of the target anatomy.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a method of diagnosis-guided image quality assessment with an NLP engine;
Fig. 2 shows three regions of interest in a clinical scan detected by the detection module;
Fig. 3 shows a clinical scan with a circular, per-feature, annotation of regional image quality;
Fig. 4a illustrates a first exemplary visualization guidance system;
Fig. 4b illustrates a second exemplary visualization guidance system; and
Fig. 5 shows a computer-implemented method of guiding clinical scan quality assessments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method of guiding clinical scan quality assessments. The method comprises identifying one or more target anatomies in a region of interest of a clinical scan of a subject and determining an indication of regional image quality for each identified target anatomy.

It has been realized that radiologists make decisions on rescan based on the diagnostic target. This is especially the case for experienced or senior radiologists. Thus, an automated system would support junior radiologists in the decision on whether they should perform a rescan or not. This could not only save the decision time for the radiologists but more importantly also save the patients from the radiation dose associated with the rescan.

A framework is proposed to guide quantitative image quality evaluation in clinical practice. This method makes more sense than a simple image scoring system because it can provide more accurate focal emphasis for the clinicians. Consequently, the users can make better decisions based on targeted diagnostic areas and avoid unnecessary rescans and recalls.

The invention described herein can provide a quick-response tool for currently inaccurate, or inefficient, image quality assessment applications, especially for junior radiologists and technologists. It could enable rescanning the patient if bad image quality is detected, while the patient is still on the couch, thus avoiding patient recalls. It could also be a gatekeeper for any unnecessary rescans which otherwise increases radiation exposure for the patients and results in inefficient use of the scanner and the radiologist and technologist's time in the clinical workflow.

While many image quality assessment systems are like a black box for the users, the framework proposed is, in essence, an explainable AI system with an optional visualization guidance system, which is reliable and convincing for the clinicians in practice.

The framework can also be used as a comprehensive training platform with enough instructions for the junior radiologists to improve their diagnosis-guided mindset, especially in the quality-oriented hospitals.

The framework is based on the idea that key aspects in making decisions on whether to acquire a rescan or not lies in understanding an exact diagnostic target and observational focus on the clinical scan.

The examples provided herein are based on the proposal to detect and assess the accurate quality of an examination using a natural language processing (NLP) engine with visual assistance to the radiologists and technologists. For example, a system may consist of an NLP engine and clinical database, a region of interest (ROI) detection module and guided segmentation module, an attention supervision module, a quality scoring module for decision making and a visualization guidance system.

For the NLP engine and clinical database, it is proposed to preferably use the technology of NLP to extract the diagnostic information from available medical notes and confirm the main diagnostic target for the exam. Through a well-designed clinical database, the related target anatomies can be inferred to subsequently focus on this specific diagnostic target.

For the ROI detection module and guided segmentation module, convolutional neural networks can be employed for the detection of an affected portion on the clinical scan and the region of interest (ROI) based on the target anatomies. When a target anatomy is detected in the ROI, it is proposed to contour all target anatomies within the ROI with the segmentation algorithms, such as outline of lung, liver, etc. Multi-organ and multi-task segmentation algorithms can be embedded in this module.

In order to make the models focus on the ROI and corrupted portions which really impact the image quality for diagnosis, it is proposed to use an attention-based model, in the attention supervision module, to learn the most important feature which most impacts the decision of whether to rescan or not. Through the supervised training strategy, a clinical knowledgebase (obtained from clinical experts) can be utilized to give a focal score prediction on the ROI and corrupted portion.

It is recognized that the real quality score in radiologists mind is not simply based on the superficial features of the clinical scan like noise level or signal noise ratio. If a quality score is based solely on these superficial features, it may misrepresent the problem of image quality of clinical scans and give out an inaccurate quality score. Hence, the diagnostic target anatomy and affected portion as described above are taken into consideration for the image quality.

A visualization interface can be used to integrate all the analysis results and give out an overall quality score. Preferably, the reasoning behind why a medical image gets a particular score is presented to the user. The interface could also give specific instructions on where a partial rescan is the best solution for the subject.

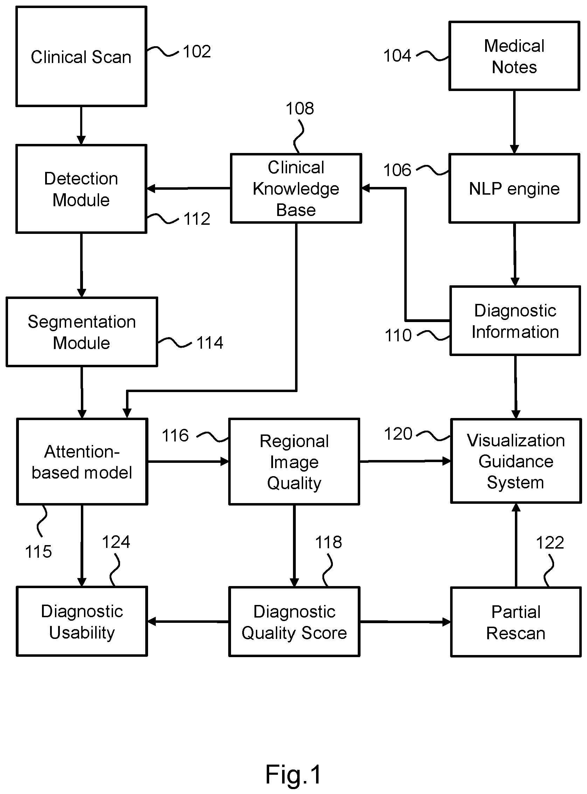

Fig. 1 shows a method of diagnosis-guided image quality assessment with an NLP engine. The starting point in the workflow is the acquisition of a clinical scan 102 and medical notes 104 of a subject.

The medical notes 104 (e.g., including discharge records and admission notes, patient metadata and clinical information) are input into an NLP engine 106 to extract diagnostic information 110 including, for example, the accurate entities of the diagnostic target anatomies and other relevant valuable information from the unstructured text data. The diagnostic information 110 can provide clinical targets of interest and/or information which enables the extraction of clinical targets of interest (e.g., a potential diagnosis).

For this purpose, an existing algorithm such as entity recognition could be used to identify target anatomies from the free text notes.

With the help of the NLP engine a crucial location and historic message can be extracted from the medical notes. For training such an algorithm, clinical experts can annotate ground truth labels using such notes.

It is further proposed to utilize expert clinical knowledge to construct a relational database 108. In this knowledge database 108, triple tuples can be used to store the required knowledge combined with the global standards of health terms such as SNOMED (Systematized Nomenclature of Medicine) and ICD (International Classification of Diseases). Briefly, the database 108 could provide all the clinical possibilities when a potential diagnosis is queried or input. The potential diagnosis can be extracted from medical notes 104 by the NLP engine 106 and form part of the diagnostic information 110.

For example, the medical notes of a subject may contain the admission note:
"34 yrs old female, Rabia received from ER on stretcher accompanied RN Sidra, with diagnosis of blunt abdominal trauma, under c/o Dr Ali. Pt had complaint of acute upper abdominal pain due to motor vehicle accidents, not relieved by taking semico at home so was brought to ER and shifted to B1 ward. Spontaneously breathing in room air with B/L equal chest movement, regular, easy breaths. Patient is conscious, awake and well oriented to time, place and person. Has IV on Right hand, no swelling or redness observed. Getting N/S @ 100cc per hour. Kept NPO. Passed urine spontaneously hematuria.

### Family involved in care."

The admission note above can be input into the NLP engine 106 which could output the clinical targets of interest (i) right upper quadrant (RUQ), (ii) perisplenic space left upper quadrant (LUQ) and (iii) pelvis (bladder). The NLP engine 106 could also output the possible diagnosis "blunt abdominal trauma". These outputs would form part of the diagnostic information 110.

In general, the diagnostic information 110 output by the NLP engine 106 can include the clinical targets of interest and key findings such as diagnostic information to be displayed by the visualization guidance system 120.

The clinical knowledge base 108 can be used to determine possible causes for the potential diagnosis such as, for the example above, blunt diaphragmatic rupture, haemorrhagic stroke, hypovolemic shock, lower genitourinary trauma, penetrating abdominal trauma in emergency medicine, pregnancy trauma and upper genitourinary trauma.

If the diagnostic information 110 does not include the clinical targets of interest, the clinical knowledge base 108 can be used to convert the available diagnostic information 110 (e.g., potential diagnosis) into clinical targets of interest. For example, when the NLP engine 106 processes the medical notes 104 to extract the diagnostic information 110, such as "Stomach Cancer with non-metastasis", this information may not be directly interpretable by the detection module 112. In these cases, the clinical knowledge base 108 can be used to obtain the clinical targets of interest. Through the clinical knowledge base 108, clinical targets of interest can be obtained from the diagnostic information 110. For example, clinical targets of interest may include: stomach, abdomen, organs around stomach etc. The clinical targets of interest could then be used to automatically trigger the detection and segmentation of these targets by the detection module 114 and the segmentation module 114 respectively.

A detection module 112 can be established to automatically detect the most important region in the clinical scan (i.e., the regions of interest, ROIs) and key landmarks using, for example, a CNN-based model and the clinical targets of interest.

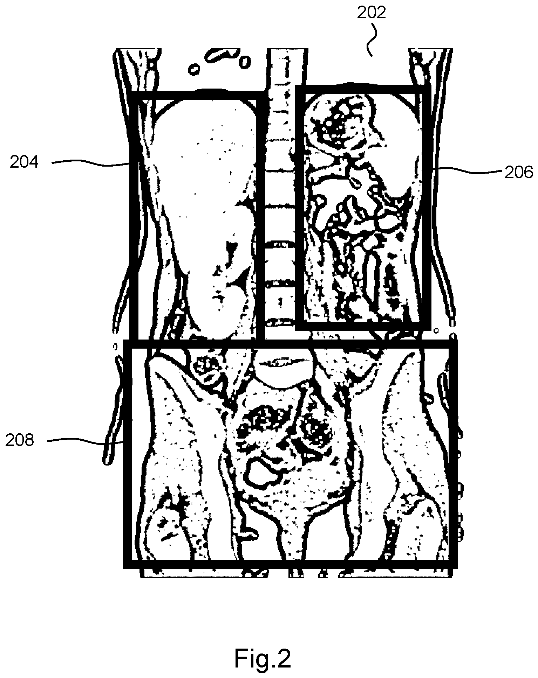

Fig. 2 shows three ROIs 204, 206 and 208 in a clinical scan 202 detected by the detection module 112. ROI 204 shows a right upper quadrant (RUQ), ROI 206 shows a left upper quadrant (LUQ), and ROI 208 shows a pelvic region. From the detected ROIs, the appropriate portion detected which covers all the possible abnormalities can be found.

Returning to Fig. 1, with the detected ROIs, corresponding multi-organ segmentation algorithms in a segmentation module 114 could be triggered to segment all the targeted anatomical features (e.g., organs) within the corresponding ROI. The segmentation algorithms in the segmentation module 114 could provide more information and accuracy for the border coverage and focal quality assessment.

The multi-organ algorithm for each ROI (e.g., right upper quadrant (RUQ), left upper quadrant (LUQ), chest, abdomen, chest-abdomen, abdomen-pelvis etc.) could be trained accordingly for the target anatomical features expected to be found in said ROIs.

The targeted anatomical features which are segmented (e.g., organs, muscles, vessels etc.) could be configured by users based on their expert judgement on the clinical indication, by using the visualization tool 120.

A regional image quality 116 can then be extracted from the target anatomical features by using trained attention-based models 115. The clinical targets of interest obtained from the diagnostic information 110 (e.g., via the clinical knowledge base 108) can also be used as inputs into the attention-based models 115. The regional image quality may include focal artifacts, complexity of the segmentation, blurring level and abnormal artifacts.

For visualization and training purposes, a scheme of circular annotation is proposed to display the regional image quality of target anatomies.

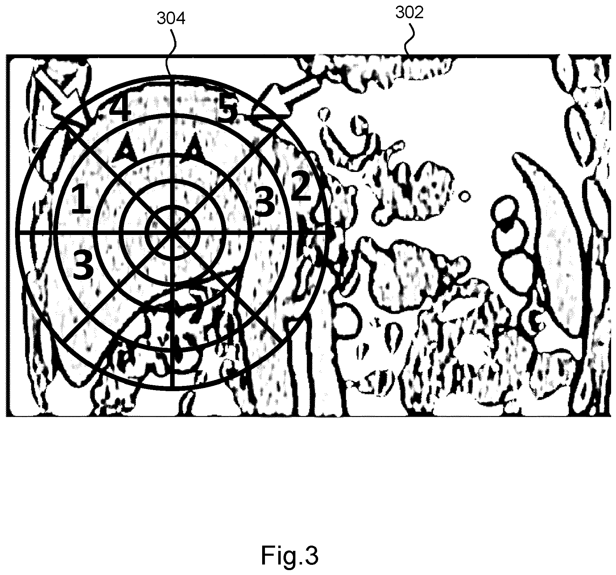

Fig. 3 shows a clinical scan 302 with a circular, per-feature, annotation 304. The circular annotation 304 comprises a plurality of portions for a target anatomical feature, where each portion can be annotated with a separate image quality score. The ground truth of image quality score (e.g., 0-5 could represent perfect to worst image quality) given by the clinical experts is annotated on the corresponding portion of the anatomical feature.

Returning to Fig. 1, the clinical knowledge base 108 may also contain the experts' annotation protocols for the supervised training of models. These protocols mainly focus on the focal annotation with the segment annotation and score annotation. A circle-like template for the annotation has been proposed here to ensure that the crucially affected part could be labelled well.

Based on the ground truth annotations from the clinical experts, a set of attention-based models 115 can be trained to learn which regions of the clinical scans are important to clinical experts such that, in use, the attention-based models 115 output regional quality scores 116 for all the targeted anatomies within the relevant ROI. Said attention-based models 115 may be convolutional neural networks (CNNs).

Using a plurality of quality score predictions for each target anatomy within the relevant ROI, an overall quality score 118, for the clinical scan can be calculated. For example, the overall quality score 118 may be in the form of an average or median value. Moreover, it is proposed to adapt different scoring strategies based on the clinical question at hand in order to make the results more sensitive for severe image quality issues.

A threshold for the overall quality score 118 can be set for the decision of whether to perform a (partial) rescan or not. Ideally, for the clinical practice within a quality-oriented hospital, it is proposed to use a score of three (out of five, where lower is better) as a threshold. If the overall quality score 118 is less than three, the diagnostic usability 124 of the clinical scan could be classified into positive diagnostic usability (i.e., indicating that the clinical scan could be used for the purpose of making a diagnosis). If the overall quality score 118 is more than three, the system could suggest that partial rescan 122 is performed. Additionally, the system may provide detailed instructions on the region for the partial rescan 122 on the visualization guidance system 120. The regional image quality scores 116 (e.g., regional circle-like scores) can be displayed on the visualization guidance system 120 to show the range of the partial rescan 122 to the user and enable the user to more easily infer the portions which may need a rescan. The visualization guidance system could then also display suggested actions (e.g., "take partial rescan from slice 5 to 50").

In general, if the overall diagnostic quality score 118 is not good enough (e.g., above/below a pre-defined threshold), then an indication of the diagnostic usability 124 should be displayed by the visualization guidance system 120 informing the user that the clinical scan 102 may not be suitable to inform a diagnosis.

If only a partial rescan is needed, instead of a full rescan, then this suggested action could be displayed by the visualization guidance system 120 as the indication of partial rescan 122.

The indication of diagnostic usability 124, and/or the indication for a partial rescan 122 can be displayed to the user by the visualization guidance system 120.

. The visualization guidance system 120 is an explainable and configurable visualization board for the users to utilize the intelligent quality system with more confidence and understanding. For example, the diagnostic information 110 can also be displayed by the visualization guidance system 120 together with the clinical scan, the detected ROIs, the regional quality scores and the overall quality score. Subject/patient information (e.g., obtained by the NLP engine 106 from the medical notes 104, or otherwise) can also be displayed by the visualization guidance system 120.

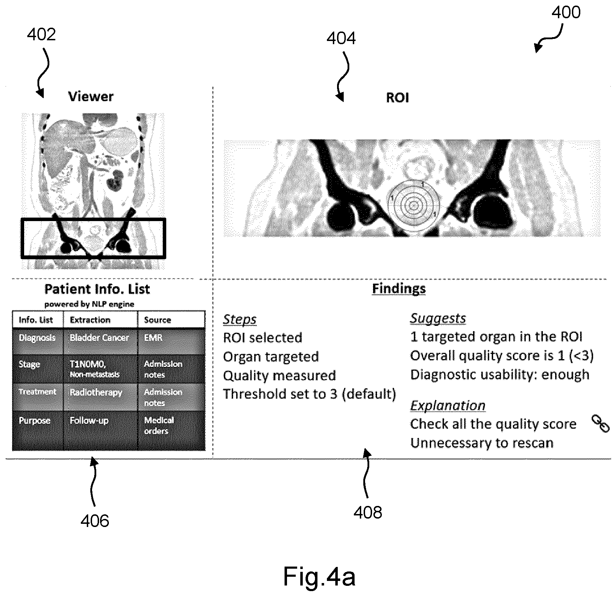

Fig. 4a illustrates a first exemplary visualization guidance system 400. The visualization guidance system 400 automatically indicates the detected ROI in the clinical scan 402 and a zoomed-in version of the ROI 404 is displayed, giving users a better focus on the target anatomies. A bounding box (or other indication of ROI) could be automatically shown on the clinical scan 402, to let the user have a better overview. Moreover, the bounding box could also be added or adjusted by the users, thus allowing them to manually choose the regions of their interest for checking and confirming.

On the other hand, the information extracted by the NLP engine can be listed on the visualization board for reference and check in a patient information portion 406 and a findings portion 408. With the help of the clinical expert knowledge database, the most related named entity for this patient could be extracted by the NLP engine from the medical notes. Users could also click a corresponding link icon to query the original medical notes.

In this example, the bounding box covers a ROI for the bladder in the clinical scan. The information portion 406 shows a diagnosis, a corresponding stage, any treatments and the purpose of the current clinical scan. The information portion 406 can also show the sources for all the information found by the NLP engine.

In the findings portion 408, all the processing steps by this system could be transparently listed. The predicted score circles could be automatically visualized on the target anatomies within the ROI 404.

Similarly, the threshold for the decision of whether to perform a rescan or not could be user-configurable. If the calculated overall quality score is less than three, then this clinical scan is appropriate for diagnosis. On the contrary, if the overall is more than three, a rescan would be suggested along with the specific range of the rescan. The exact suggestion of rescan range could be calculated by the detection module of the quality system.

The regional image quality scores displayed on the score circles provide an explanation to the user as to why a particular overall quality score was given or why a rescan may be needed. In this example, the regional quality scores for the bladder circle are sufficient and no rescan is required.

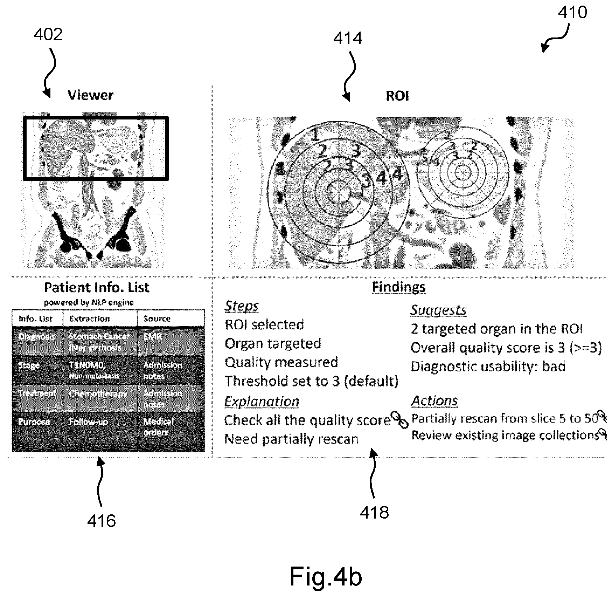

Fig. 4b illustrates a second exemplary visualization guidance system 410. In this example, the same clinical scan 402 as shown in Fig. 4a is used, but a different ROI is selected. The relevant ROI 414 corresponds to the stomach and the liver shown in the clinical scan. The information portion 416 shows that the relevant diagnoses are stomach cancer and liver cirrhosis, and the findings portion 418 shows that the overall quality score is three. Even though the clinical scan 402 is the same, the regional quality scores for the liver and the stomach are insufficient and thus the overall score of three indicates that a partial rescan should be performed.

The visualization guidance system has the advantage that it acts as a timely alarm for insufficient image quality when patient is still on the couch and a gatekeeper for unnecessary rescans. Moreover, the visualization guidance system is advantageous in that it provides outputs to the user providing the reasonings behind the proposed evaluation. This could help clinicians make better and more informed decisions in real clinical practice whilst saving unnecessary dose and time.

The visualization guidance system could also be utilized for clinical training as it contains the whole understandable procedure of diagnosis-guided decision-making in the visualization guide. Especially for junior radiologists in quality-oriented hospitals, the explainable and understandable guidance system can be used to establish their principles of real judgement on image quality. Moreover, this visualization guidance system could also be empowered with other functions to improve the clinical workflow in the radiology department. For example, when partial rescan suggestions are received with an exact rescan range from this system, the visualization guidance system could be used to further fuse the two series of clinical scans for readout. Otherwise, the radiologist would still have to read two image series individually.

In the example above, the visualization system shows the circle shaped image quality scoring. It is also proposed to adopt the shape of the visualizer based on the shape of the organ in question. AI based techniques could be employed to detect the organ and modify the shape of the scoring template to oval or rectangular shape. Similarly, different templates could be proposed using AI for the same organ depending on the case at hand. For example, for the normal sized liver, the system could be configured to the circle shaped template, which could be changed to oval if an enlarged liver is detected by AI. In other use cases, the visualization guide could be configured to indicate slice-wise scores.

Of course, in a basic implementation, a single regional quality score can be provided for each target anatomical feature.

An additional feature for the visualization guidance system could include actions or recommendations upon a user's click. Additionally, in the case of longitudinal studies or multiple exams of the same subject, the system could also be configured to review all the available scans for that subject and potentially help the user to spot the differences between exams easily.

In practical implementations, this system could also be adjusted to accept the key information of a diagnostic target from the manually input by clinicians.

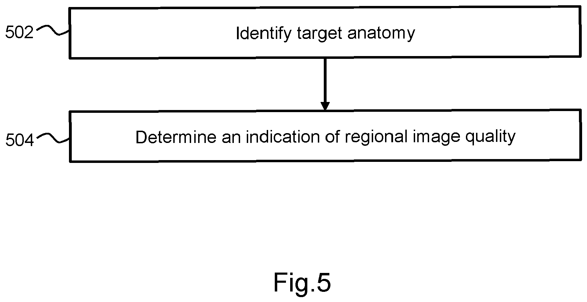

Fig. 5 shows a computer-implemented method of guiding clinical scan quality assessments. The method comprises identifying one or more target anatomies in a region of interest of a clinical scan of a subject in step 502. The target anatomies (i.e., target anatomical features) may include organs, muscles, bones, vessels etc. The region of interest may be determined by a detection module as described above. The region of interest may also be set by the user. A clinical scan is generally a medical image and may be, for example, an ultrasound scan, an x-ray scan, an MRI scan, a CT scan etc.

After the target anatomies are identified, an indication of regional image quality is determined for each identified target anatomy in step 504. The indication of regional image quality for a target anatomy may be in the form of one or more scores of image quality for the target anatomy (e.g., as described above with the circular annotations). Preferably, a plurality of regional quality scores are provided for each target anatomy, wherein each regional quality score corresponds to a different portion of the target anatomy.

An indication of regional image quality is based on the image quality of a portion of the clinical scan corresponding to a target anatomy. Thus, the indication of regional image quality provides the image quality for the important portions of the clinical scan.

An attention-based model can be trained to determine the indications of regional image quality. A proposed training process for the attention-based model has two stages using a supervised learning strategy. In the first stage, clinical experts would provide regional quality scores for clinical scans based on clinical targets. The regional quality scores are mapped onto the clinical scans using, for example, a circle annotation. In the second stage, it is proposed to utilize segmentation results and corresponding features extracted from the clinical scans as additional training data.

It is further proposed to use data fusion and post-processing to generate heat maps (attention-based feature map) corresponding to clinical scans using the afore-mentioned training data. These feature maps provide an indication of which regions of the clinical scan the clinical experts give the most attention to.

During training, the attention-based model takes as inputs the regional quality scores (e.g., the circle annotation), the segmentation results and the feature maps to compute a set of attention weights for each spatial location in the feature maps. These attention weights are used to compute a weighted sum of the feature maps to produce a single attention map that highlights the most important/salient regions of a clinical. Thus, in use, the trained attention-based model can take a clinical scan as the input and output regional quality scores for salient portions of the clinician scan.

In summary, the attention-based model is trained to focus on the most important/salient regions of clinical scans. This is achieved by incorporating clinical knowledgebase and expert opinion to guide the focal quality assessment during training. This approach can improve the accuracy and robustness of the model, especially in cases where the dataset is limited or noisy.

Preferably, a plurality of regional image quality scores are determined for each target anatomy, wherein each regional image quality score corresponds to a different portion of the target anatomy in the clinical scan.

Any method described herein may be computer-implemented, where computer-implemented means that the steps of said methods are performed by one or more computers and where a computer is defined as a device suitable for processing data. A computer may be suitable for processing data according to prescribed instructions.

The skilled person would be readily capable of developing a processor for carrying out any herein described method. Thus, each step of a flow chart may represent a different action performed by a processor and may be performed by a respective module of the processing processor.

One or more steps of any of the methods described herein may be performed by one or more processors. A processor comprises an electronic circuit suitable for processing data.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

A single processor or other unit may fulfill the functions of several items recited in the claims.

The computer program carrier may comprise computer memory (e.g., random-access memory), computer storage (e.g., hard drives, solid-state drives etc.). The computer program carrier may be a bitstream carrying the computer program code.

A computer program code may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method of guiding clinical scan quality assessments, the method comprising:
identifying (502) one or more target anatomies in a region of interest of a clinical scan of a subject; and
for each identified target anatomy, determining (504) an indication of regional image quality.

2. The method of claim 1, further comprising displaying a portion of the clinical scan corresponding to the region of interest overlayed with the indications of regional image quality and further displaying subject information relating to the subject and/or diagnostic information indicative of a potential diagnosis for the subject.

3. The method of claims 1 or 2, further comprising determining the region of interest by:
extracting a diagnostic target from medical notes of the subject;
determining possible causes for the diagnostic target; and
determining the region of interest based on the possible causes.

4. The method of any of claims 1 to 3, further comprising determining a scan quality score for the clinical scan by combining the indications of regional image quality.

5. The method of claim 4, further comprising, based on the scan quality score, classifying the clinical scan into suitable or unsuitable for diagnostic use.

6. The method of any of claims 1 to 5, wherein the indication of regional image quality comprises one or more of:
a detection of an artifact;
a type of artifact identified;
a complexity of identifying the corresponding target anatomy; and
blurring of the target anatomy.

7. The method of claim 6, further comprising inputting the clinical scan into a neural network configured to output one or more of:
the detection of an artifact;
the type of artifact identified;
the complexity of identifying the corresponding target anatomy; and
blurring of the target anatomy.

8. The method of any of claims 1 to 7, wherein the region of interest is user adjustable.

9. A computer program carrier comprising computer program code which, when executed on a computer, causes the computer to perform all of the steps according to any of claims 1 to 8.

10. A system of guiding clinical scan quality assessments, the system comprising a processor configured to:
identify (502) one or more target anatomies in a region of interest of a clinical scan of a subject; and
for each identified target anatomy, determine (504) an indication of regional image quality.

11. The system of claim 10, wherein the processor is further configured to:
display a portion of the clinical scan corresponding to the region of interest overlayed with the indications of regional image quality; and
display subject information relating to the subject and/or diagnostic information indicative of a potential diagnosis for the subject.

12. The system of claims 10 or 11 wherein the processor is further configured to determine the region of interest by:
extracting a diagnostic target from medical notes of the subject;
determining possible causes for the diagnostic target; and
determining the region of interest based on the possible causes.

13. The system of any of claims 10 to 12, wherein the processor is further configured to determine a scan quality score for the clinical scan by combining the indications of regional image quality.

14. The system of any of claims 10 to 13, wherein the indication of regional image quality comprises one or more of:
a detection of an artifact;
a type of artifact identified;
a complexity of identifying the corresponding target anatomy; and
blurring of the target anatomy.

15. The system of claim 14, wherein the processor is further configured to input the clinical scan into a neural network configured to output one or more of:
the detection of an artifact;
the type of artifact identified;
the complexity of identifying the corresponding target anatomy; and
blurring of the target anatomy.
